# EUROPÄISCHE PATENTANMELDUNG

(11) **EP 0 593 959 A1**
(43) Veröffentlichungstag der Anmeldung: **27.04.1994**
(21) Anmeldenummer: 93115853.9
(22) Anmeldetag: 30.09.1993
(51) Int. Cl.: A61K 7/043

(54) **Bindemittel für wässrige Nagellacke**

(30) Priorität: 13.10.1992 DE 4234417
(71) Anmelder: Hüttenes-Albertus Chemische-Werke GmbH, D-40549 Düsseldorf (DE)
(72) Erfinder: Hellwig, Jürgen, Dr. rer. nat. Dipl.-Chem., D-29308 Winsen/Aller (DE); Van Sande, Jan, Dipl.-Ing., D-41749 Viersen (DE)
(74) Vertreter: Eikenberg, Kurt-Rudolf

(57) **Zusammenfassung**

Es handelt sich um ein in Wasser dispergiertes Bindemittel (Filmbildner) für wässrige Nagellacke, die einen Gehalt an üblichen weiteren Zusätzen aufweisen. Das Bindemittel umfaßt ein urethanisiertes ölmodifiziertes Polyesterharz in wässriger Dispersion mit einem Feststoffgehalt von 30 bis 50 % das vorzugsweise in einer Menge von 20 bis 90 Gew.-%, bezogen auf den fertigen Nagellack eingesetzt wird. Der Nagellack kann dabei noch bis zu 60-Gew.%, bezogen auf den fertigen Nagellack, an einem wasserverdünnbaren oder wasserlöslichen natürlichen oder synthetischen Harz, insbesondere Cellulosenitrat, enthalten.

Das Bindemittel zeichnet sich durch höchst wünschenswerte Eigenschaften aus, insbesondere durch hohe Kratzfestigkeit und geringe Wasserquellbarkeit bei gutem Glanz und gutem Verlauf.

Die Herstellung des Bindemittels erfolgt zweckmäßig durch Umsetzung eines ölmodifizierten Polyesterharzes mit einer Dihydroxycarbonsäure und einem Diisocyanat im NCO/OH-Verhältnis von 2 : 1 bis 1 : 1 in Gegenwart eines zumindest partiell mit Wasser mischbaren Lösungsmittels und anschließende Abdestillation des Lösungsmittels.

## Beschreibung

Die Erfindung betrifft ein in Wasser dispergiertes Bindemittel (Filmbildner) für Nagellacke mit einem Gehalt an üblichen weiteren Zusätzen, die nachfolgend auch als "wässriger Nagellack" oder "wasserverdünnbarer Nagellack" bezeichnet werden.

Die in der Praxis gebräuchlichen Nagellacke wurden bisher nahezu ausschließlich auf der Basis physikalisch trocknender Bindemittel hergestellt, die in organischen Lösungsmitteln gelöst wurden. Insbesondere Cellulosenitrat in Kombination mit Weichmachern und gegebenenfalls weiteren Kunstharzen spielte hierbei eine dominierende Rolle. In der Regel enthalten diese Nagellacke noch Farbstoffe und Pigmente sowie Hilfsmittel zur Verbesserung der anwendungstechnischen und optischen Eigenschaften.

In jüngster Zeit ist nun das Umweltbewußtsein ständig gewachsen, was auch zu Konsequenzen für die Kosmetik-Industrie geführt hat. Die hohen Lösungsmittelanteile der konventionellen Nagellacke sind umweltbelastend und auch gesundheitlich nicht ganz unbedenklich. Daher hat es nicht an Versuchen gefehlt, die organischen Lösungsmittel durch Wasser zu ersetzen.

So sind in der DE-A 39 11 262 wäßrige Nagellacke auf der Basis von Polyurethanen und/oder Polyurethan-Acrylat-Copolymeren beschrieben, die normalerweise noch Zusätze enthalten, beispielsweise wasserlösliche Harze (z.B. Acrylat-Styrol-Harze) zur Beeinflussung der Film-Eigenschaften, modifizierte Silikone zur Erhöhung der Härte und Kratzfestigkeit, Verdickungsmittel, Trocknungsbeschleuniger, Sedimentationsverhinderer sowie nagelpflegende und nagelschützende Substanzen. Diese Nagellacke genügen jedoch insbesondere wegen ihrer geringen Kratzfestigkeit und Härte, aber auch wegen anderer Eigenschaften wie z.B. einer starken Wasserquellbarkeit noch nicht den praktischen Bedürfnissen.

Auch in der DE-A 39 31 237 sind wäßrige Nagellacke auf der Basis von lösungsmittelfreien aliphatischen Polyurethandispersionen und/oder Acrylatdispersionen - mit weiteren Zusätzen - vorgestellt. Die Kratzfestigkeit soll in diesem Fall durch Aziridin als Vernetzungsmittel verbessert werden. Der Einsatz von Aziridin im kosmetischen Bereich ist aber zunehmend umstritten, insbesondere wegen der carcinogenen Wirkung dieses Stoffes.

Es besteht also nach wie vor ein Bedarf an einem Bindemittel für wässrige Nagellacke mit verbesserten Eigenschaften, die den Bedürfnissen der Praxis gerecht werden. Ein solches Bindemittel soll mit der Erfindung zur Verfügung gestellt werden.

Dieses Ziel erreicht die Erfindung durch ein Bindemittel, das ein urethanisiertes ölmodifiziertes Polyesterharz in wässriger Dispersion mit einem Feststoffgehalt von 30 bis 50 % umfaßt. Vorzugsweise wird dieses Bindemittel in einer Menge von 20 bis 90 Gew.-%, bezogen auf den fertigen Nagellack eingesetzt.

Die Erfindung geht von der Erkenntnis aus, daß die Mängel der bisher bekannten wässrigen Bindemittel-Dispersionen im wesentlichen durch das Polyurethan-Grundgerüst der Bindemittel bedingt sind und deshalb kaum durch Zusätze zum Bindemittel beseitigt werden können. Daher verläßt die Erfindung den bisher eingeschlagenen Weg und sieht stattdessen ein Bindemittel mit einem völlig anderen Grundgerüst vor, nämlich ein Harz mit einem Polyester-Grundgerüst, das zusätzlich ölmodifiziert und urethanisiert ist.

Das Ausgangsprodukt für das erfindungsgemäß vorgesehene Bindemittel ist ein kurzöliges bis mittelöliges Alkydharz, das auf herkömmliche Weise erhalten werden kann, zweckmäßig durch Veresterung von di- und polyfunktionellen Alkoholen mit Dicarbonsäuren bzw. deren Anhydriden in Gegenwart von höheren Fettsäuren. An Dicarbonsäuren bzw. Dicarbonsäure-Anhydriden haben sich beispielsweise Phthalsäureanhydrid, Isophthalsäure, Terephthalsäure, Adipinsäure, Maleinsäureanhydrid, Dimerfettsäure und deren Mischungen als gut geeignet erwiesen. An Polyolen kommen beispielsweise Pentaerythrit, Dipentaerythrit, Glycerin, Trimethylolethan, Trimethylolpropan, Ethylenglykol, Propylenglykol, Neopentylglykol einzeln oder in Mischung miteinander in Betracht. Als Fettsäuren sind beispielsweise Sojaölfettsäure, Kokosölfettsäure, Saflorölfettsäure, Leinölfettsäure, isomerisierte Fettsäuren und Dienfettsäuren wie Ricinenfettsäuren und andere natürliche und synthetische Fettsäuren einsetzbar, wiederum einzeln oder in Mischung. Die natürlichen Fettsäuren können dabei auch als Triglyceride eingesetzt werden, wobei sie dann über Umesterungsreaktionen eingebaut werden. Im übrigen können die Fettsäuren auch noch mit anderen Monocarbonsäuren, wie z.B mit Benzoesäure und Alkylbenzoesäuren gemischt sein.

Durch das Polyester-Grundgerüst in Verbindung mit der Öl-Modifikation werden wesentliche anwendungstechnische Eigenschaften des erfindungsgemäß vorgesehenen Bindemittels vorbestimmt, insbesondere eine geringe Wasserquellbarkeit und eine hohe Kratzfestigkeit. Es läßt sich aber durch Auswahl des Verhältnisses Polyol/Carbonsäure/Fettsäure auch die Härte und Elastizität ohne Einbuße an Kratzfestigkeit und ohne Erhöhung der Wasserquellbarkeit von "sehr elastisch" bis "extrem hart" einstellen, wodurch das erfindungsgemäße Bindemittel optimal auf unterschiedliche Anwendungsgebiete, z.B. für Farblacke, für Klarlacke, für Grundierlacke oder für Nagelverfestiger eingestellt werden kann. Dabei kann "hart" als Normalfall angesehen werden kann, während "elastisch" in erster Linie für überlange künstliche Nägel und "extrem hart" für Nagelverfestiger in Betracht kommt. In diesem Zusammenhang sei hervorgehoben, daß elastische und dennoch hochkratzfeste wässrige Nagellacke bislang nicht für darstellbar gehalten wurden, weil bei den bisher bekannten wässrigen Nagellacken Härte und Kratzfestigkeit parallel gehen.

Allerdings sind ölmodifizierte Polyesterharze im allgemeinen für die Zwecke der Erfindung nicht hydrophil genug, weshalb die Harze in weiterem Verfolg des Erfindungsgedankens noch mit zusätzlichen hydrophilen Gruppen, insbesondere neutralisierten Carboxylgruppen versehen werden. Demgemäß sieht die Erfindung einen zusätzlichen, hier als "Urethanisierung" bezeichneten Arbeitsgang vor, bei dem die ölmodifizierten Polyesterharze (genauer: deren freie OH-Gruppen) noch mit einer Dihydroxycarbonsäure und einem Diisocyanat umgesetzt werden. Das NCO/OH-Äquivalentverhältnis liegt dabei etwa im Bereich von 2 : 1 bis 1 : 1, d.h. das Diisocyanat wird vorzugsweise mit Überschuß eingesetzt. Hierdurch werden Carboxylgruppen in das Polyester-Grundgerüst eingefügt, und außerdem werden weitere Vernetzungen bewirkt, nämlich einmal über Urethan-Bindungen zwischen den NCO-Gruppen und den OH-Gruppen, und zum anderen über Harnstoff-Bindungen, die sich wegen des Überschusses an Diisocyanaten in Gegenwart von Wasser ergeben. Die erfindungsgemäß vorgesehene Urethanisierung verändert das Polyester-Grundgerüst nicht, sondern ergänzt es nur durch zusätzliche Hydrophilie und eine zusätzliche Vernetzung.

Die Urethanisierung durch Umsetzung des ölmodifizierten Polyesterharzes mit einer Dihydroxycarbonsäure und einem Diisocyanat erfolgt zweckmäßig in Gegenwart von Lösungsmitteln, die völlig oder partiell mit Wasser mischbar sind, vorzugsweise Aceton und Methylethylketon. Als Dihydroxycarbonsäure ist dabei insbesondere Dimethylolpropionsäure bevorzugt. Für die Neutralisation der Carboxylgruppe kommen tertiäre Amine wie Trimethyl-, Triethyl- und Triphenylamin zum Einsatz. Als Diisocyanat können aliphatische, cycloaliphatische oder aromatische Diisocyanate verwendet werden, beispielsweise Hexamethylendiisocyanat, Trimethylhexamethylendiisocyanat, 4.4'-Dicyclohexylmethandiisocyanat, Isophorondiisocyanat, 4.4'-Diisocyanato-diphenylmethan, 2.4-Diisocyanato-toluol oder 2.6-Diisocyanato-toluol und deren Mischungen. Die Reaktion wird viskositätsmäßig gesteuert, bei Gutbefund wird Wasser zugesetzt. Nach Abdestillieren des Lösungsmittels erhält man eine stabile wässrige Dispersion des urethanisierten ölmodifizierten Polyesterharzes.

Das erfindungsgemäße Bindemittel bildet sofort nach dem Abdunsten des Wassers einen klebfreien harten Lackfilm, der bereits weitestgehend die an einen Nagellack gestellten Anforderungen erfüllt. Der besondere Vorteil besteht aber darin, daß mit und nach der physikalischen Antrocknung, ohne daß ein besonderes Vernetzungsmittel erforderlich ist, eine oxidative Vernetzung der ungesättigten Fettsäurereste stattfindet, die zu einer außerordentlichen Steigerung der Kratzfestigkeit des Lackfilm führt. Des weiteren ist hervorzuheben eine hohe Brillanz und Fülle dieses Bindemittels.

Diese oxidative Vernetzung und damit die Langzeit-Haltbarkeit des gebildeten Lackfilms läßt sich noch durch Zusatz von Sikkativen (Trockenstoffen, meist in Form von Metallsalzen organischer Säuren) steigern. Allerdings sind Sikkative nur bedingt einsetzbar, weil sie die Entfernbarkeit des Nagellacks ungünstig beeinflussen und dadurch einen der weiteren wesentlichen Vorteile des erfindungsgemäßen Bindemittels beeinträchtigen können. Dieser weitere wesentliche Vorteil des erfindungsgemäßen Bindemittels besteht nämlich darin, daß der ausgehärtete Lackfilm mit Alkoholen, wie z.B. Ethanol entfernt werden kann, während sich sowohl die konventionellen lösungsmittelhaltigen Nagellacke als auch die bisher bekannten wässrigen Nagellacke auf Basis von Polyurethandispersionen nur mit weniger umweltverträglichen Stoffen wie Aceton und/oder Estern (z.B. Amylacetat) entfernen lassen. Daher wird der Zusatz von Sikkativen zweckmäßig gering gehalten.

Vorteilhaft kann das erfindungsgemäße Bindemittel noch einen Zusatz von natürlichen und/oder synthetischen wasserlöslichen oder wasserverdünnbaren Harzen enthalten, beispielsweise von Schellack, Kautschuk, Polyurethanharzen, Acrylharzen, Melaminharzen, Epoxidharzen und Acrylat-Styrol-Harzen. Dieser Zusatz kann entweder mit dem erfindungsgemäßen Bindemittel vorgemischt oder gemeinsam mit dem erfindungsgemäßen Bindemittel dem Nagellack zugesetzt sein, wobei die Zusatzmenge bis zu 60 Gew.-%, bezogen auf den fertigen Nagellack, beträgt. Besonders zweckmäßig wird zu diesem Zweck ein in Wasser dispergiertes Cellulosenitrat mit einem Feststoffgehalt von 25 - 50 % eingesetzt, wodurch der Nagellack zusätzliche vorteilhafte Eigenschaften in Richtung auf die konventionellen lösungsmittelhaltigen Nagellacke erhält.

Das erfindungsgemäße Bindemittel ist mit allen weiteren Bestandteilen handelsüblicher Nagellacke verträglich., Zu erwähnen sind hierbei Verdicker in Form von Cellulosederivaten, Polysacchariden, Silikaten (Tone und Montmorillonite) und synthetischen Polymerisaten wie Acrylat- und Polyurethan-Verdickern, die normalerweise in wässriger Dispersion angewandt werden. Im Rahmen der Erfindung sind dabei besonders solche Verdicker vorteilhaft, die dem Nagellack thixotrope Eigenschaften verleihen, beispielsweise Umsetzungsprodukte von Heteropolysacchariden mit einem aminofunktionellen Silan, die in der eigenen EP-B 287 589 beschrieben sind und in einer Menge von 0,1 bis 3,0 Gew.%, bezogen auf den fertigen Nagellack, eingesetzt werden können.

Im Falle eines Farblacks enthalten Nagellacke noch ein Farbmittel in Form von Farbstoffen und/oder Pigmenten, einschließlich Perlglanzpigmenten oder Fischsilber für besondere Farbeffekte. Diese Farbmittel können auch bei dem erfindungsgemäßen Bindemittel in den üblichen Mengen ohne weiteres zum Einsatz kommen.

Auch alle sonstigen gängigen Bestandteile von handelsüblichen Nagellacken, wie beipielsweise Trocknungsbeschleuniger, Sedimentationsverhinderer, Netz- und Dispergiermittel, pH-Regulatoren, Oberflächenadditive, Filmbildungshilfsmittel, Konservierungsstoffe, Füllstoffe sowie nagelpflegende und nagelschützende Substanzen, ergeben in Verbindung mit dem erfindungsgemäßen Bindemittel keinerlei Probleme.

Die Erfindung wird durch die nachfolgenden Beispiele näher erläutert:

### BEISPIEL 1

Dieses Beispiel beschreibt die Herstellung eines urethanisierten ölmodifizlerten Polyesterharzes, das sich für die Zwecke der Erfindung gut bewährt hat.

In einem 4-Liter Rundkolben, der mit Rührer, Thermometer und Azeotropaufsatz ausgerüstet ist, werden 275 g Sojaölfettsäure, 80 g Benzoesäure, 154 g Pentaerythrit und 160 g Phthalsäureanhydrid unter Zugabe von Xylol als Schleppmittel bei 220 °C verestert. Die Reaktion wird abgebrochen, wenn das Harz eine Säurezahl unter 8 und bei 50 %iger Verdünnung in Xylol eine Auslaufzeit von mindestens 30 sec (4 mm DIN-Becher) besitzt.

In einem nachfolgenden Schritt wird dieses ölmodifizierte Polylyesterharz mit 62 g Dimethylolpropionsäure und 248 g Isophorondiisocyanat sowie 420 g Methylethylketon und 43 g Triethylamin bei ca. 85 °C umgesetzt, bis eine mit Methylethylketon auf 35 % Feststoffgehalt verdünnte Probe eine Auslaufzeit von etwa 60 sec hat. Es wird nun auf 60 °C gekühlt und 1450 g destilliertes Wasser zugesetzt. Danach wird noch etwa 3 h bei 50 °C gehalten und dann bei dieser Temperatur das Lösungsmittel abdestilliert.

Man erhält eine stabile wäßrige Dispersion eines urethanisierten ölmodifizierten Polyesterharzes mit einem Festkörpergehalt von 40 % und einer Viskosität von 120 mPa.s. Diese Dispersion ergibt einen Lackfilm von gutem Glanz, guter Haftung und hoher Härte.

### BEISPIEL 2

Dieses Beispiel beschreibt die Herstellung eines weiteren urethanisierten ölmodifizierten Polyesterharzes, das sich für die Zwecke der Erfindung ebenfalls gut bewährt hat.

In einem 4-Liter-Rundkolben, der mit Rührer, Thermostat und absteigendem Kühler ausgerüstet ist, werden 83 g Phthalsäureanhydrid, 158 g Isophthalsäure , 67 g Diethylenglykol, 413 g Saflorölfettsäure und 135 g Glycerin im Schmelzverfahren verestert. Das resultierende Harz hat eine Säurezahl von 5 und bei 80 %iger Verdünnung in Methylethylketon eine Auslaufzeit von 55 sec (4 mm DIN-Becher).

In einem nachfolgenden Schritt erfolgt die Umsetzung mit 61 g Dimethylolpropionsäure, 433 g Isophorondiisocyanat, 270 g Methylethylketon und 38,5 g Triethylamin. Wenn die Viskosität einer mit Methylethylketon auf 50 % Feststoffgehalt verdünnten Probe eine Auslaufzeit von mindestens 200 sec erreicht, wird auf 50 °C gekühlt und 1610 g destilliertes Wasser zugesetzt. Dann wird weitere 3 h bei dieser Temperatur gerührt und schließlich unter vermindertem Druck das Lösungsmittel abdestilliert.

Man erhält eine stabile wäßrige Dispersion eines urethanisierten ölmodifizierten Polyesterharzes mit einem Festkörpergehalt von 45 % und einer Viskosität von 160 mPa.s. Diese Dispersion ergibt ebenfalls einen Lackfilm von gutem Glanz, guter Haftung und hoher Härte.

### BEISPIEL 3

Die wässrige Dispersion gemäß Beispiel 1 wird mit einem Acrylat-Styrol-Copolymer vermischt, und zwar im Verhältnis
60 Gewichtsteile Polyesterharzdispersion 40 % in Wasser
25 Gewichtsteile Acrylat-Styrol-Copolymer 42 % in Wasser.

Diese Mischung ergibt einen Lackfilm, der sich durch hohe Härte, hohen Glanz, guten Verlauf sowie eine verbesserte Wirtschaftlichkeit auszeichnet.

### BEISPIEL 4

Dieses Beispiel beschreibt Bindemittel auf der Basis einer Polyesterdispersion gemäß Beispiel 1 mit einem Zusatz an Cellulosenitrat, welches als ergänzendes Harz in einer wässrigen Dispersion vorliegt. Es werden vermischt
80 Gewichtsteile Polyesterharzdispersion 40 % in 38 % Wasser
25 Gewichtsteile Cellulosenitratdispersion 38 % in Wasser
Diese Mischung ergibt einen Lackfilm, der bei gutem Glanz, guter Haftung, gutem Verlauf und hoher Härte besonders elastisch ist.

## Patentansprüche

1. In Wasser dispergiertes Bindemittel (Filmbildner) für wässrige Nagellacke, die einen Gehalt an üblichen weiteren Zusätzen aufweisen, umfassend ein urethanisiertes ölmodifiziertes Polyesterharz in wässriger Dispersion mit einem Feststoffgehalt von 30 bis 50 %.

2. Bindemittel nach Anspruch 1, mit einem zusätzlichen Gehalt von bis zu 60 Gew.-%, bezogen auf den fertigen Nagellack, an einem wasserverdünnbaren oder wasserlöslichen natürlichen oder synthetischen Harz, insbesondere einer wässrigen Cellulosenitrat-Dispersion.

3. Verfahren zur Herstellung des Bindemittels gemäß Anspruch 1 durch Umsetzung eines ölmodifizierten Polyesterharzes mit einer Dihydroxycarbonsäure und einem Diisocyanat im NCO/OH-Verhältnis von 2 : 1 bis 1 : 1 in Gegenwart eines zumindest partiell mit Wasser mischbaren Lösungsmittels und anschließende Abdestillation des Lösungsmittels.

4. Verwendung einer wässrigen Dispersion von urethanisierten ölmodifizierten Polyesterharzen als Bindemittel von Nagellacken, und zwar in einer Menge von 20 bis 90 Gew.-%, bezogen auf den fertigen Nagellack.
